# EUROPÄISCHE PATENTANMELDUNG

(11) **EP 4 663 157 A1**
(43) Veröffentlichungstag der Anmeldung: **17.12.2025**
(21) Anmeldenummer: 25182647.5
(22) Anmeldetag: 13.06.2025
(51) Int. Cl.: A61C 8/00

(54) **VERFAHREN ZUM HERSTELLEN EINES DENTALIMPLANTATS**

(30) Priorität: 13.06.2024 DE 102024116671
(71) Anmelder: medentis medical GmbH, 53474 Bad Neuenahr-Ahrweiler (DE)
(72) Erfinder: SCHOLZ, Alexander, 53474 Bad Neuenahr-Ahrweiler (DE)
(74) Vertreter: Müller Schupfner & Partner Patent- und Rechtsanwaltspartnerschaft mbB (Muc)

(57) **Zusammenfassung**

Bereitgestellt wird ein Verfahren zum Herstellen eines Dentalimplantats, umfassend die Schritte
(a) Bereitstellen eines Basiskörpers für ein Dentalimplantat aus einem Basiswerkstoff, der eine Oberfläche aufweist,
(b) Reinigen der Oberfläche des Basiskörpers,
(c) Aufbringen einer nanokristallinen TiO₂-Schicht auf zumindest einen Teil der Oberfläche des Basiskörpers durch Sputtern, wobei während des Sputterns oder im Anschluss daran ein Erwärmungsschritt stattfindet.

## Beschreibung

Die Erfindung betrifft ein Verfahren zum Herstellen eines Dentalimplantats, bei dem durch Sputtern eine TiO₂-Schicht auf einen Basiskörper aufgebracht wird und diese anschließend erwärmt wird. Ferner betrifft die Erfindung ein Dentalimplantat, erhältlich nach diesem Verfahren.

Dentalimplantate werden seit vielen Jahren erfolgreich eingesetzt und weisen in der Regel einen Basiskörper aus Titan oder einem Keramikwerkstoff auf, wobei Titan der vorwiegend eingesetzte Basiswerkstoff ist. Solche Dentalimplantate haben sich in Langzeitstudien bewährt. Die Basiskörper von Dentalimplantaten weisen in der Regel einen Verankerungsbereich auf, zum Beispiel ein Schraubgewinde, einen Halsbereich, der den Übergang vom Knochen zum Zahnfleisch und von dort zum Mundraum darstellt, und einen Aufbaubereich, auf den ein Prothetikelement wie beispielsweise eine Brücke oder eine Krone aufgeschraubt oder zementiert werden kann.

Bekannt sind bereits Implantate mit einer Oberflächenbeschichtung aus Titandioxid (TiO₂), das die feste Integration in den umgebenden Knochen (Osseointegration) verbessert. Bekannte Dentalimplantate mit einer TiO₂-Oberfläche weisen jedoch den Nachteil auf, dass die Sicherheit der Osseointegration noch verbesserungsbedürftig ist. Insbesondere ist der Anteil der Modifikation Anatas der TiO₂-Beschichtung zu gering für eine Photoaktivierung, wobei eine Photoaktivierung die Osseointegration durch eine Hydrophilisierung verbessern würde.

Aufgabe der Erfindung ist daher ein Verfahren zum Herstellen eines Dentalimplantats und ein danach erhältliches Dentalimplantat bereitzustellen, mit dem eine möglichst gute Osseointegration erreicht werden kann.

Diese Aufgabe wird gelöst durch ein Verfahren zum Herstellen eines Dentalimplantats gemäß Patentanspruch 1 und ein nach dem Verfahren erhältliches Dentalimplantat gemäß Patentanspruch 11. Weitere Merkmale, Ausführungsformen sowie Vorteile ergeben sich aus den abhängigen Ansprüchen und der Beschreibung.

Ein Aspekt der Erfindung betrifft ein Verfahren zum Herstellen eines Dentalimplantats, umfassend die Schritte
(a) Bereitstellen eines Basiskörpers für ein Dentalimplantat aus einem Basiswerkstoff, der eine Oberfläche aufweist,
(b) Reinigen der Oberfläche des Basiskörpers,
(c) Aufbringen einer nanokristallinen TiO₂-Schicht auf zumindest einen Teil der Oberfläche des Basiskörpers durch Sputtern, wobei
   entweder während des Aufbringens der TiO₂-Schicht der Basiskörper auf 150 - 300 °C erwärmt wird und/oder
(d) nach dem Aufbringen der TiO₂-Schicht, der Basiskörper, einschließlich der TiO₂-Schicht, für mindestens 2 h auf 150 - 500 °C erwärmt wird.

Überraschenderweise wird durch das oben genannte Erwärmen das nanokristalline TiO₂ praktisch vollständig in die Modifikation Anatas überführt, wodurch eine UV-Aktivierung der TiO₂-Schicht möglich ist und so eine sehr gute Osseointegration erreicht wird.

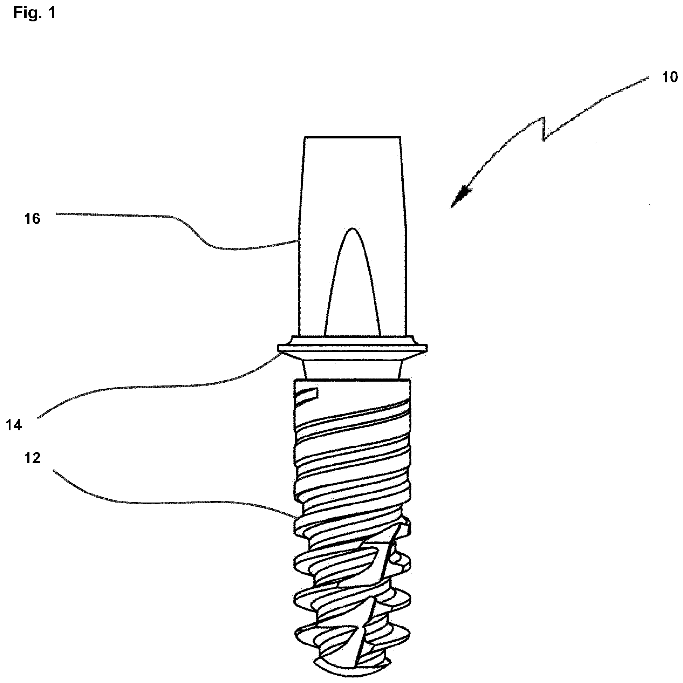

In Fig. 1 ist ein erfindungsgemäßes Dentalimplantat 10 gezeigt. Das Implantat 10 weist einen Verankerungsbereich 12 auf, der zur Verankerung im Kieferknochen vorgesehen ist, hier in Form eines Schraubgewindes. An den Verankerungsbereich 12 schließt sich der Halsbereich 14 an und daran der Aufbaubereich 16. Der Aufbaubereich 16 ist zur Aufnahme eines Prothetikelements vorgesehen, beispielsweise einer Krone oder einer Brücke.

Im erfindungsgemäßen Verfahren wird nach dem Bereitstellen zunächst eine Reinigung der Oberfläche des Basiskörpers durchgeführt, vorzugsweise ein Plasmareinigen, weiter vorzugsweise eine Plasmaoberflächenreinigung und Plasmapolitur des Basiskörpers unter Vakuum.

Die Titandioxid-Oberflächenbeschichtung wird erfindungsgemäß durch Sputtern auf den Basiskörper aufgebracht, vorzugsweise durch einen gepulsten Sputterprozess. Ein derartiger Sputterprozess ist grundsätzlich bekannt aus der DE 10 2004 024 351 A1 sowie aus O. Zywitzki et al., "Structure and Properties of Crystalline Titanium Oxide Layers Deposited by Reactive Pulse Magnetron Sputtering", in Surface and Coatings Technology, 180-181 (2004) 538-543.

Durch das Sputtern entsteht eine nanokristalline TiO₂-Schicht. Die Titandioxidschicht, die durch Sputtern aufgebracht wird, weist an der Oberfläche eine natürliche Nanorauigkeit auf, die durch eine nanokristalline Struktur des Titandioxids erzeugt wird. Die Nanorauigkeit verbessert die spätere Osseointegration des Dentalimplantats. Beim Sputtern wird üblicherweise in einer Vakuumanlage gearbeitet und so können hochreine Schichten erzeugt werden. Die nanokristalline TiO₂-Schicht besteht in der Regel aus verschiedenen Modifikationen, wobei der Anteil an Anatas und Rutil über die Prozessparameter des Sputterns gesteuert, d.h. erhöht bzw. erniedrigt werden kann. Allerdings haben die Experimente im Rahmen der vorliegenden Erfindung gezeigt, dass der Anatas-Anteil selbst unter optimalen Bedingungen für ein Aufsputtern von Anatas noch zu niedrig für eine wirksame spätere Photoaktivierung ist. Eine Photoaktivierung kann die Osseointegration durch eine Hydrophilisierung der TiO₂-Beschichtung nochmals deutlich verbessern.

Daher wird erfindungsgemäß durch einen Erwärmungsschritt (Tempern) entweder während des Sputterns oder im Anschluss an das Sputtern der Anteil an Anatas erhöht und insbesondere so auch erreicht, dass eine UV-Aktivierung (Photoaktivierung mit UV-Licht) möglich wird. Durch die Bestrahlung mit UV-Licht wird die vorwiegend Anatas umfassende TiO₂-Schicht stark hydrophil und an der Oberfläche und antibakteriell. Durch die Hydrophilität wird die Osseointegration verbessert, weil ein besseres Einwachsen in den Knochen ermöglicht wird. Dies erhöht die Sicherheit der Osseointegration, d. h. der Anteil der eingewachsenen Dentalimplantate erreicht einen höheren Prozentsatz.

In einer bevorzugten Ausführungsform des erfindungsgemäßen Verfahrens ist der der Basiswerkstoff, aus dem der Basiskörper besteht, aus Titan, einer Titanlegierung oder einer Keramik. Die Keramik ist bevorzugt Zirkonoxid. Weiter vorzugsweise ist der Basiswerkstoff aus Titan oder Zirkonoxid. Am meisten bevorzugt ist als Basiswerkstoff aus Titan, insbesondere Titan Grade 4.

Unter Titan Grade 4 wird wie üblich eine Titanlegierung verstanden, umfassend
≤ 0,50 Gew.-% Fe,
≤ 0,08 Gew.-% C,
≤ 0,40 Gew.-% O,
≤ 0,05 Gew.-% N,
≤ 0,015 Gew.-% H,
jeweils bezogen auf das Gesamtgewicht der Titanlegierung,
und den Rest Ti.

In einer bevorzugten Ausführungsform des erfindungsgemäßen Verfahrens wird zwischen dem Reinigen der Oberfläche des Basiskörpers (Schritt (b)) und dem Aufbringen einer nanokristallinen TiO₂-Schicht (Schritt (c)) keine weitere Schicht auf den Basiskörper aufgebracht. Die TiO₂-Schicht befindet sich somit in dieser bevorzugten Ausführungsform unmittelbar auf der Oberfläche des Basiskörpers und damit unmittelbar auf dem Basiswerkstoff.

In einer bevorzugten Ausführungsform des erfindungsgemäßen Verfahrens wird der Basiskörper während des Sputterns auf 170 - 250 °C erwärmt, weiter vorzugsweise auf 180 - 220 °C. Das Erwärmen nach dem Aufbringen der TiO₂-Schicht erfolgt vorzugsweise auf 180 - 480 °C, weiter vorzugsweise auf eine Temperatur von 200 - 480 °C und am meisten bevorzugt auf eine Temperatur von 350 - 470 °C, insbesondere 450 °C. Das Erwärmen nach dem Aufbringen der TiO₂-Schicht erfolgt vorzugsweise für mindestens 2,5 h, weiter vorzugsweise für mindestens 3 h, insbesondere für 3 - 12 h. Das Erwärmen nach dem Aufbringen der TiO₂-Schicht erfolgt vorzugsweise unmittelbar im Anschluss an das Sputtern, weiter vorzugsweise innerhalb von 60 Minuten nach Ende des Sputterns.

Nach dem Erwärmungsschritt, wobei entweder während des Aufbringens der TiO₂-Schicht der Basiskörper auf 150 - 300 °C erwärmt wird und/oder nach dem Aufbringen der TiO₂-Schicht, der Basiskörper, einschließlich der TiO₂-Schicht, für mindestens 2 h auf 150 - 500 °C erwärmt wird, liegt bevorzugt ≥ 99 Gew.-% des TiO₂, bezogen auf das Gesamtgewicht der TiO₂-Schicht, in der Modifikation Anatas vor. Besonders bevorzugt liegt nach dem Erwärmen das TiO₂ in der TiO₂-Schicht, bezogen auf das Gesamtgewicht der TiO₂-Schicht, zu ≥ 99,5 Gew.-%, insbesondere ≥ 99,9 Gew.-% in der Modifikation Anatas vor.

Das Sputtern des erfindungsgemäßen Verfahrens ist vorzugsweise ein Magnetronsputtern.

Das Aufbringen der TiO₂-Schicht erfolgt auf zumindest einem Teil der Oberfläche des Basiskörpers. Der Basiskörper weist vorzugsweise einen Verankerungsbereich auf, insbesondere ein Schraubgewinde, zur Verankerung des Dentalimplantats im Knochen, einen Halsbereich und einen Aufbaubereich, zur Aufnahme des Prothetikelements. Das Aufbringen der TiO₂-Schicht erfolgt in einer bevorzugten Ausführungsform des erfindungsgemäßen Verfahrens zumindest im Verankerungsbereich, insbesondere auf einem Schraubgewinde des Basiskörpers. Die TiO₂-Schicht kann auch auf weiteren Bereichen des Basiskörpers aufgebracht werden.

Die erfindungsgemäße TiO₂-Schicht auf dem Basiskörper hat vorzugsweise eine mittlere Schichtdicke von 10 - 1000 nm (Nanometer), bevorzugt 100 - 300 nm. Unter einer mittleren Schichtdicke wird im Sinne der Erfindung der arithmetische Mittelwert aus mindestens drei, vorzugsweise fünf, gemessenen Schichtdicken verstanden. Dazu wird an mindestens drei, vorzugsweise fünf, zufällig ausgewählten Stellen die Schichtdicke gemessen, beispielsweise unter einem Mikroskop in einem Schnitt senkrecht zur Oberfläche. Anschließend wird der arithmetische Mittelwert der gemessenen Schichtdicken ermittelt.

Weiter ist es bevorzugt, dass nach dem Schritt des Erwärmens im Halsbereich und/oder im Aufbaubereich eine TiO₂-Schicht aufgebracht wird, die zu mehr als 80 Gew.-%, bezogen auf das Gesamtgewicht der TiO₂-Schicht, aus der Modifikation Rutil besteht. Weiter ist es bevorzugt, dass diese TiO₂-Schicht zu mehr als 90 Gew.-%, insbesondere zu mehr als 95 Gew.-% aus der Modifikation Rutil besteht, jeweils bezogen auf das Gesamtgewicht der TiO₂-Schicht. Diese TiO₂-Schicht im Halsbereich und/oder im Aufbaubereich weist vorzugsweise keine Nanorauigkeit auf.

Bevorzugt wird eine TiO₂-Schicht, die überwiegend aus Anatas besteht, im sogenannten Unipolarmodus abgeschieden und eine Rutilschicht bevorzugt im sogenannten Bipolarmodus abgeschieden.

In einer bevorzugten Ausführungsform des erfindungsgemäßen Verfahrens wird beim Sputtern Stickstoffgas (N₂) hinzugefügt. Dadurch wird Stickstoff in die TiO₂-Schicht eingebaut, wodurch das Anatas nach dem Erwärmen durch sichtbares Licht, insbesondere Licht einer Wellenlänge zwischen 400 und 780 nm, photoaktivierbar wird. Ohne erfindungsgemäß daran gebunden zu sein, wird angenommen, dass durch den Einbau von Stickstoff in die TiO₂-Schicht (Zudotieren von Stickstoff) die Bandlücke des TiO₂ verändert wird, sodass die Schicht auch durch die Einwirkung von sichtbarem Licht photoaktivierbar wird. Dies vereinfacht die Photoaktivierung, insbesondere in der Praxis, da so beim Zahnarzt vor dem Einsetzen des Implantats nicht mit UV-Licht gearbeitet werden muss.

Die vorliegende Erfindung betrifft auch ein Dentalimplantat, erhältlich nach dem erfindungsgemäßen Verfahren. Dieses Dentalimplantat umfasst einen Basiskörper für ein Dentalimplantat und darauf aufgebracht eine nanokristalline TiO₂-Schicht, zumindest auf einem Teil der Oberfläche des Basiskörpers, aufgebracht durch Sputtern, wobei die nanokristalline TiO₂-Schicht, bezogen auf das Gesamtgewicht der TiO₂-Schicht, zu ≥ 99 Gew.-% in der Modifikation Anatas vorliegt. Vorzugsweise liegt die TiO₂-Schicht, bezogen auf das Gesamtgewicht der TiO₂-Schicht, ≥ 99,5 Gew.-%, insbesondere ≥ 99,9 Gew.-% in der Modifikation Anatas vor.

Es versteht sich, dass die vorstehend genannten und die nachfolgend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen. Die genannten Vorteile von Merkmalen oder von Kombinationen mehrerer Merkmale sind lediglich beispielhaft und können alternativ oder kumulativ zur Wirkung kommen. Die Kombination von Merkmalen unterschiedlicher Ausführungsformen der Erfindung oder von Merkmalen von unterschiedlichen Patentansprüchen ist abweichend von den gewählten Rückbezügen der Patentansprüche möglich.

Im Folgenden wird die Erfindung anhand von zwei Beispielen weiter erläutert:

### Beispiele

### Beschichtungsprozesses ohne Substratheizung

Implantate werden stehend auf dem Objektträger vorbereitet.

Kompressor einschalten und Vakuum-Pumpen starten.

Wenn Vakuum in der Beschichtungskammer vorliegt, die markierten Objektträger auf Palette legen und in die Transferkammer einschleusen. Transferkammer abpumpen.

Argon in die Beschichtungskammer einlassen, weiter unter Vakuum, Hochspannung an die Kathode anlegen (4 kW) und Oberfläche der Kathode für 5 Minuten sputtern. Anschließend die Hochspannung ausschalten.

Palette in die Beschichtungskammer einschleusen.

Anschließend Objektträger und Substrat unter die Kathode fahren, Gas (Ar / O₂, 1:1) in die Beschichtungskammer einlassen.

Pulser einschalten und Hochspannung an die Kathode anlegen (4 kW) und ca. 10 Minuten beschichten. Anschließend wird die Hochspannung ausgeschaltet und der Objektträger ausgeschleust.

Anschließend die Dentalimplantate in einen Temperofen überführen und auf ca. 350 bis 470 °C heizen, nach ca. 2 bis 5 h den Ofen ausschalten und abkühlen lassen. Anschließend werden die Dentalimplantate entnommen.

### Beschichtungsprozesses mit Substratheizung

Durchführung wie oben, mit dem Unterschied, dass die Substratheizung vor dem Einschleusen auf eine Temperatur von 150 bis 300 °C gebracht wird.

Nach dem Ausschleusen des Objektträgers aus der Beschichtungskammer endet dann der Beschichtungsprozess, ein anschließendes Erhitzen ist nicht mehr notwendig.

### Bezugszeichenliste:

- 10: Dentalimplantat
- 12: Verankerungsbereich
- 14: Halsbereich
- 16: Aufbaubereich

## Patentansprüche

1. Verfahren zum Herstellen eines Dentalimplantats (10), umfassend die Schritte
(a) Bereitstellen eines Basiskörpers für ein Dentalimplantat (10) aus einem Basiswerkstoff, der eine Oberfläche aufweist,
(b) Reinigen der Oberfläche des Basiskörpers,
(c) Aufbringen einer nanokristallinen TiO₂-Schicht auf zumindest einen Teil der Oberfläche des Basiskörpers durch Sputtern, **dadurch gekennzeichnet, dass**
entweder während des Aufbringens der TiO₂-Schicht der Basiskörper auf 150 - 300 °C erwärmt wird und/oder
(d) nach dem Aufbringen der TiO₂-Schicht, der Basiskörper, einschließlich der TiO₂-Schicht, für mindestens 2 h auf 150 - 500 °C erwärmt wird.

2. Verfahren nach Anspruch 1, **dadurch gekennzeichnet, dass** nach dem Erwärmen ≥ 99 Gew.-% des TiO₂, bevorzugt ≥ 99,9 Gew.-% des TiO₂, bezogen auf das Gesamtgewicht der TiO₂-Schicht, in der Modifikation Anatas vorliegt.

3. Verfahren nach Anspruch 1 oder 2, **dadurch gekennzeichnet, dass** der Basiswerkstoff aus Titan, einer Titanlegierung oder einer Keramik ist.

4. Verfahren nach einem der vorangehenden Ansprüche, **dadurch gekennzeichnet, dass** der Basiswerkstoff aus Titan oder Zirkonoxid ist.

5. Verfahren nach einem der vorangehenden Ansprüche, **dadurch gekennzeichnet, dass** der Basiswerkstoff aus Titan Grade 4 ist.

6. Verfahren nach einem der Ansprüche 1 bis 4, **dadurch gekennzeichnet, dass** der Basiswerkstoff eine Titanlegierung ist, umfassend
≤ 0,50 Gew.-% Fe,
≤ 0,08 Gew.-% C,
≤ 0,40 Gew.-% O,
≤ 0,05 Gew.-% N,
≤ 0,015 Gew.-% H,
jeweils bezogen auf das Gesamtgewicht der Titanlegierung,
und den Rest Ti.

7. Verfahren nach einem der vorangehenden Ansprüche, **dadurch gekennzeichnet, dass** zwischen Schritt (b) und Schritt (c) keine weitere Schicht auf den Basiskörper aufgebracht wird.

8. Verfahren nach einem der vorangehenden Ansprüche, **dadurch gekennzeichnet, dass** der Basiskörper während des Sputterns auf 180 - 220 °C erwärmt wird.

9. Verfahren nach einem der vorangehenden Ansprüche, **dadurch gekennzeichnet, dass** das Erwärmen nach dem Aufbringen der TiO₂-Schicht auf 350 - 460 °C für mindestens 2,5 h erfolgt, bevorzugt für 3 - 12 h.

10. Verfahren nach einem der vorangehenden Ansprüche, **dadurch gekennzeichnet, dass** der Basiskörper einen Verankerungsbereich (12) aufweist, vorzugsweise ein Schraubgewinde, zur Verankerung des Dentalimplantats (10) im Knochen, einen Halsbereich (14) aufweist und einen Aufbaubereich (16), zur Aufnahme eines Prothetikelements, aufweist und das Aufbringen der TiO₂-Schicht zumindest im Verankerungsbereich (12) stattfindet.

11. Verfahren nach Anspruch 10, **dadurch gekennzeichnet, dass** nach dem Schritt des Erwärmens im Halsbereich (14) und/oder im Aufbaubereich (16) eine TiO₂-Schicht aufgebracht wird, die zu mehr als 90 Gew.-%, bezogen auf das Gesamtgewicht der TiO₂-Schicht, aus der Modifikation Rutil besteht.

12. Verfahren nach einem der vorangehenden Ansprüche, **dadurch gekennzeichnet, dass** die TiO₂-Schicht eine mittlere Schichtdicke von 100 - 300 nm aufweist.

13. Verfahren nach einem der vorangehenden Ansprüche, **dadurch gekennzeichnet, dass** das Sputtern ein Magnetronsputtern ist.

14. Verfahren nach einem der vorangehenden Ansprüche, **dadurch gekennzeichnet, dass** beim Sputtern Stickstoffgas hinzugefügt wird, so dass Stickstoff in die TiO₂-Schicht eingebaut wird.

15. Dentalimplantat (10), erhältlich nach einem Verfahren gemäß einem der Ansprüche 1 bis 14.
